# EUROPEAN PATENT SPECIFICATION

(11) **EP 4 299 132 B1**
(45) Date of publication and mention of the grant of the patent: **15.07.2026**
(21) Application number: 23211263.1
(22) Date of filing: 29.08.2020
(51) Int. Cl.: A61K 38/39, A61K 36/288, A61K 36/22, A61K 36/53, A61K 36/79, A61K 36/36, A61K 9/48, A61K 9/14, A61K 9/08, A61P 31/12

(54) **PHARMACEUTICAL COMPOSITION CONTAINING A SURFACTANT AND NATURAL PLANT EXTRACTS FOR PREVENTING AND TREATING CORONAVIRUS**
PHARMAZEUTISCHE ZUSAMMENSETZUNG MIT EINEM TENSID UND NATÜRLICHEN PFLANZENEXTRAKTEN ZUR VORBEUGUNG UND BEHANDLUNG VON CORONAVIRUS
COMPOSITION PHARMACEUTIQUE CONTENANT UN TENSIOACTIF ET DES EXTRAITS NATURELS DE PLANTES POUR LA PRÉVENTION ET LE TRAITEMENT DU CORONAVIRUS

(43) Date of publication of application: 03.01.2024
(62) Divisional of application: 20951308.4
(73) Proprietor: Portillo Rosado, Rosa Maria, 11108 Santo Domingo (DO)
(72) Inventor: Portillo Rosado, Rosa Maria, 11108 Santo Domingo (DO)
(74) Representative: Stolmár & Partner Intellectual Property GmbH

(56) References cited:
- CN-A- 111 298 049
- PATRA JAYANTA KUMAR ET AL: "): Chemical compounds, antiviral properties, and clinical relevance", vol. 34, no. 6, 29 January 2020 (2020-01-29), GB, pages 1248 - 1267, XP093011632, ISSN: 0951-418X, Retrieved from the Internet <URL:https://onlinelibrary.wiley.com/doi/full-xml/10.1002/ptr.6614> DOI: 10.1002/ptr.6614
- QUEIROZ MARYL S. ET AL: "Cytokine Profile and Natural Killer Cell Activity in Listeria Monocytogenes Infected Mice Treated Orally with Petiveria Alliacea Extract", vol. 22, no. 3, 27 January 2000 (2000-01-27), US, pages 501 - 518, XP093011641, ISSN: 0892-3973, Retrieved from the Internet <URL:http://dx.doi.org/10.3109/08923970009026008> DOI: 10.3109/08923970009026008
- ZEFZOUFI MANAL ET AL: "Composition of essential oil of Moroccan Dysphania ambrosioides and its antimicrobial activity against bacterial and fungal phytopathogens", vol. 102, no. 1, 1 February 2020 (2020-02-01), IT, pages 47 - 58, XP093011645, ISSN: 1125-4653, Retrieved from the Internet <URL:http://link.springer.com/article/10.1007/s42161-019-00371-x/fulltext.html> DOI: 10.1007/s42161-019-00371-x

## Description

### FIELD OF INVENTION

The present invention relates to pharmaceutical compositions for the treatment and prevention of the coronavirus based on a surfactant with degreasing properties, the procedure for obtaining said compositions, as well as said compositions for the treatment and prevention of coronavirus for oral administration in the form of capsules or drinkable ampoules, and for intramuscular administration.

### BACKGROUND OF THE INVENTION

The coronavirus is one of the viruses that is wreaking the most havoc across the world population.

Much is said about it, but until now we have not achieved a medicine or a vaccine capable of preventing and controlling it.

Every day new expectations of studies appear, but none that we can affirm with property this is what stops it.

The damage caused to both humanity and the economy is incalculable.

But we cannot drink a shampoo or a cleansing gel due to its harmful effects on health, and its consequences, but that would be a solution.

One of the great inconveniences that arise is that there are times when the coronavirus does not present any symptoms, which is the main danger since a large group of people can be infected without even knowing it.

Given the death rate from this disease, saving time is a must.

A trial with a completely natural formula can reduce the risks to a minimum.

Patent CA2569080A from 2006 refers to the field of coronavirus and molecules for its prevention.

Patent CN100500162C requested by TOAGOSEI CO LTD in 2003 provides effective management of the coronavirus and can be converted into the various antiviral agent of many cleaning products, it contains a silver ion carrier antiviral agent that may inactivate the virus, but selected of chemical compound. Patent CN105687226A from 2016 deals with a preparation to inhibit coronavirus infections having Astragalus as the active ingredient.

Patent JP2003384125A from 2003 deals with an aerosol disinfectant preventing infectious disease, either by air or by direct treatment into the hands, its active ingredient is hinokitiol, aloe vera, green tea, albomarginata and houttuynia cordata, it is sprayed and said to prevent the spread of infection.

Patent CN111298049A relates to a traditional Chinese medicine composition for treatment of COVID-19, and application thereof. The traditional Chinese medicine component is prepared from radix bupleuri, Scutellaria baicalensis, rhizomapinellinae praeparata, cassia twigs, official magnolia flowers, aster, folium isatidis, radix isatidis, dandelion, honeysuckle flowers, fructus forsythia, chrysanthemum, honeysuckle stems, rhizomaphragmitis, lalang grass rhizome, fructus viticis and fried coix seeds. The composition has functions of dispelling mild diaphoresis and relieving exterior syndromes and clearing away heat and removing toxicity. Furthermore, it has significant curative effects when being applied to treatment of suspected cases and mild cases of COVID-19 as well as prevention of people with a direct or indirect contact history.

Tests pose complex ethical situations, but for these unusual situations, we will have to look for unusual solutions as well.

It is known that the people with the lowest risk of death or developing a severe form of the disease are young people without pre-existing health conditions.

Older people are the most at risk. The mortality rate increases exponentially from the age of 65, approximately.

In China, for example, the mortality rate of infected people up to 40 years old is 0.2%, but among those between 70 and 79 years old it increases up to 8%, while from 80 years old and the number increases up to 14.8%.

Fortunately, so far, no children have died from coronavirus.

Evidently, there are children who are also infected with the virus, but they do not get sick, or only show slight symptoms.

Much has been said about the causes, but nothing that gives an answer to it yet.

### DESCRIPTION OF THE INVENTION

The present invention aims to develop a pharmaceutical formulation obtained by combining the powdered extract obtained from aqueous extracts of different plants and a surfactant with degreasing properties as active ingredients, without side effects from its intake.

More particularly, the present invention has as its object the formulation of different pharmaceutical dosage forms such as, according to the invention, capsules, drinkable ampoules, and injectable solution ampoules, as well as non-claimed tea, that comprise non-hydrolyzed collagen as a surfactant, (soap), powdered extracts of Dandelion (Taraxacum officinale), Anamu (Petiveria Alliacea), Epazote (Dysphania ambrosioides), Oregano, (Origanum vulgare), Star Anise, (Illicium verum), together with preservatives for the treatment of patients suffering from coronavirus, surprisingly obtaining a notable improvement 24 hours after starting treatment, and it has been possible to verify an integral and significant improvement at 48 hours after applying the administration regimen.

This objective of the invention was solved as set out in the appended set of claims.

### DETAILED DESCRIPTION OF THE INVENTION

Currently there is no pharmaceutical formula that contains a surfactant with degreasing properties as a base and that can be ingested at the same time.

We have achieved a formula that contains a surfactant with degreasing properties and can be taken without side effects, with the benefit of preventing the virus from surviving. We are facing a great solution for the world.

So far, the only thing that has been shown to stop and delay its spread is soap, because coronaviruses are wrapped in a lipid layer that soap inhibits.

It is known that washing body parts, Ex. washing hands and hard surfaces, countertops and sinks can significantly decrease the population of microorganisms including pathogens. Coronaviruses have a lipid membrane that gives the particle relatively low stability, especially when compared to the naked particle of the common cold virus. That makes it relatively easy to remove them from your hands using soapy solutions.

Therefore, protection seems simple, social distance and use of soap.

Surfactants have antibacterial properties that is why they can eliminate microorganisms, due to their cleaning power.

What we can see is that there is a relationship with the production and decrease of collagen. When collagen is at its maximum production, the incidence is very slight, but it increases as well as its production decreases, which is why it attacks the elderly more strongly since they do not produce collagen at that age.

We know that collagen is the most abundant protein in our body, the main component of the tissues that make up various parts of the body, including tendons, ligaments, gums, teeth, skin and muscles.

As a result of the tests carried out, we were able to verify that when using plant extracts with non-hydrolyzed collagen for their surfactants and degreasing power, (as detailed in patent P-2019-0173 filed in 2019 by Rosa María Portillo and in the process of approval) when ingested, the spread of the coronavirus is inhibited, since the formulation, having this surfactant and the molecule not being hydrolyzed, is larger in size and makes its absorption much slower, being able to inhibit the virus in the intestinal tract. Since the only thing that inhibits it so far is soap, when ingesting the formulation it destroys the lipid layer of the virus, when mixed with plant extracts, it potentiates the effect of the other elements.

Which comes to solve the problem that we are experiencing at the moment, in this case a product of covid-19, since this would be the only pharmaceutical formulation that contains a degreaser in its formulation and that can be ingested without side effects, either in one capsule, one drinkable ampoule, one injectable ampoule. Furthermore, in a not claimed embodiment, the formulation may be in the form of a tea for the elderly.

The fundamental ingredients that are combined in the composition, as well as the functions that they fulfill within the composition are the following:
Non-hydrolyzed collagen:
   It is a natural active ingredient and a key protein in the human body, not hydrolyzed it is a surfactant with degreasing and cleansing properties, and as it is not hydrolyzed the molecule is larger which will make the process of crossing the intestinal barrier slower, with which can inhibit the virus in the intestinal tract.
Dandelion:
   It contains high levels of the antioxidant beta-carotene, which provides strong protection against cell damage and oxidative stress, prevents bleeding in the liver, and contributes to blood cleansing. It protects liver tissue, and has diuretic properties widely used in kidney problems.
Anamu:
   It is analgesic and anti-inflammatory in various skin conditions.
Epazote
   It is a natural antiparasitic, has calming and relaxing properties.
   It is used to treat stomach problems such as diarrhea, vomiting, and stomach pain. Helps treat flu and cough.
Oregano:
   With properties for conditions of the respiratory system with congestion, due to its anti-inflammatory, analgesic and antiseptic effect and it has an antimicrobial effect.
Star anise:
   Used for spasms of the stomach and gallbladder. With antibacterial and fungicidal active ingredients, used to treat flu, bronchitis, cough and asthma.
Sodium benzoate:
   preservative
Potassium sorbate:
   Preservative.

The qualitative and quantitative composition by weight of the active ingredients, solvents and preservatives proposed in this formulation, as well as their function within the composition, is as follows:

| | | |
|---|---|---|
| Non-hydrolyzed collagen | 1%-35% | Surfactants |
| Dandelion extract | 5%-30% | Active ingredient |
| Epazote extract | 3%-20% | Active ingredient |
| Oregano extract | 3%-20% | Active ingredient |
| Star anise extract | 5%- 25% | Active ingredient |
| Sodium benzoate | 1% -15% | Preservative |
| Potassium sorbate extract | 0.25% | Preservative |
| Treated wáter | 15%-19.5% | Solvent |

### PROCEDURE FOR THE CAPSULES ELABORATION

The procedure for preparing these capsules includes several stages:
1. In the first stage of the process, the different raw materials are prepared, which entails the inspection, classification, and approval of the raw material to be used in the production of the final product, which will reach a final product in powder form. which is filled into hard gelatin capsules.
2. After the raw materials have been approved in the raw material reception area, they go to the pre-production area where they are prepared for entry into the production area. It is in this area where the shell is removed from those raw materials that require it, as well as the seeds, are cleaned and rinsed with treated water from the treatment plant.
3. When the raw materials leave this area, they enter the production area where they enter a stainless-steel mixer with temperature control, which is fed with steam from a Pyro-tubular boiler, which allows the temperature to be increased. in the same. In this mixer we add all the raw materials of the formula except for the non-hydrolyzed collagen and the preservatives, because these must be added in the final stage of the process at a temperature of 30°C to 40°C to avoid their degradation by temperature and then they are damage the product over time.
   In this mixer, the different raw materials, all mixed within it, are subjected to a temperature of 96°C (205°F), followed by periods of agitation every 5 minutes, with the aim that at that temperature the water can absorb the properties and nutrients from each of the raw materials present in the mixer, this will allow us to obtain a well-charged liquid, rich in all the properties from the herbs, fruits and raw materials that were subjected in this part of the production process.
4. The resulting liquid that comes out of the mixer now goes to another piece of equipment called a pressure filter. It is in this equipment that even the smallest solid particle that may be mixed with the liquid extract is separated. The objective in this part of the process is to obtain a liquid free of solid particles. At this stage we will take the opportunity to add the collagen and the Preservatives.
5. The filtered liquid passes to a Spray Dryer ADL 311 type spray dryer or spray dryer. At this stage of the process, what is achieved is that the liquid from the pressure filter turns into powder. The feed of the concentrated and filtered liquid is fed by means of a peristaltic pump with a silicone hose, this spray equipment has a temperature system, spray system at the tip of the outlet nozzle, blower, heater, suction filter and exhaust filter, through which it performs the drying and subsequent spraying of the liquid, converting it into powder.
6. The powder obtained in the Pulverization equipment now goes to a NJP 1200D Encapsulating Machine, which is an automatic machine that allows the powdered product to be encapsulated in Gelatin Capsules, which can be #0 Gelatin Capsules, for example, in #0 Red/Black hard gelatin capsules from ACG Associated Capsules Pvt Ltd.
7. Once the product is encapsulated, these capsules will now go to a piece of equipment called the SP-G100 Capsule Packing Machine. The objective to be achieved in this equipment is to place the capsules in the final package, which will serve as a vehicle to make get the product to the final consumer.

The capsules thus obtained according to the described process have a storage stability at ambient conditions of 2 years.

### Example 1.

With the capsules obtained according to example 1, patients diagnosed with coronavirus shall be prescribe for 7 days with a dosage of three capsules per day with a composition of ingredients as follows:

| | |
|---|---|
| Non-hydrolyzed collagen | 25% |
| Dandelion extract | 20% |
| Anamu extract | 10% |
| Epazote extract | 15% |
| Oregano extract | 19.5% |
| Star anise extract | 10% |
| Sodium benzoate | 0.25% |
| Potassium sorbate | 0.25% |

### PROCEDURE FOR THE PREPARATION OF TEA BAGS (not claimed)

The homogeneous powder obtained according to steps 1 to 5 previously described in the procedure for obtaining the capsules is now packed in bags or sacks of porous paper, silk or nylon, similar to those used in tea bags from which it is prepared. An infusion with hot water useful in the treatment of coronavirus. The bags obtained according to the described process have a storage stability at ambient conditions of 2 years.

### Example 2 (not claimed).

### Based on my personal experience:

I was the first model for the study, the symptoms began, I felt a dry cough, shortness of breath, diarrhea, body aches, tiredness. The rash all over my belly and back I knew I was infected, I took the test, it was positive. I am a high-risk patient, 60 years old, high blood sugar, high blood pressure, too many factors against me. I prepared the formula mentioned below, I refrained from taking any medication, only my formula, surprisingly the symptoms had begun to disappear within hours, the pain went away, I began to breathe normally, the diarrhea disappeared, the hardest thing was the rash on the body, which was completely eliminated within two days of taking the formula.

I only had to drink for 7 days, 2 cups a day, of the following composition of ingredients:

| | |
|---|---|
| Non-hydrolyzed collagen | 30% |
| Dandelion | 15% |
| Anamu | 15% |
| Epazote | 17% |
| Oregano | 15% |
| Star anise extract | 7.50% |
| Sodium benzoate | 0.25% |
| Potassium sorbate | 0.25% |

### PROCEDURE FOR THE PREPARATION OF DRINKABLE AMPOULES

The procedure for the preparation of drinkable ampoules consists of the following steps:
1. In the first stage of the process, the different raw materials are received at the plant, which will go through an inspection, classification, and approval process to be used in the production of our final product, which will be drinkable ampoules.
2. After the raw materials have been approved in the raw material reception area, they go to the pre-production area where they are prepared for entry into the production area. It is in this area where the shell is removed from those materials. Raw materials that require it, as well as the seeds, are cleaned and rinsed with treated water from the treatment plant.
3. When the raw materials leave this area, they enter the production area where the first equipment or unit operation they enter is a stainless-steel mixer with temperature control, which is fed with steam, which allows to achieve the increase of the temperature in it. In this mixer we add all the raw materials of our formula, except the non-hydrolyzed collagen and the preservatives, because these must be added at a temperature of 30°C to 40°C to avoid their degradation due to high temperatures and that later they do not can have the expected effect on the final product.
4. In this mixer, the different raw materials, all mixed within it, are subjected to a temperature of 96°C (205°F), followed by periods of agitation every 5 minutes, with the aim that at that temperature the water can absorb the properties and nutrients of each of the raw materials present in the mixer, this will allow us to obtain a well-charged liquid, rich in all the properties from the herbs, fruits and raw materials that were subjected in this part of the production process.
5. The resulting liquid that comes out of the mixer now goes to another piece of equipment called a pressure filter. It is in this equipment that even the smallest solid particle that may be mixed with the liquid extract is separated. The objective in this part of the process is to obtain a liquid free of solid particles. At this stage we will take the opportunity to add the non-hydrolyzed collagen and the preservatives.
6. The container chosen to store the final product is the container: Special Ampoules for Drinkable Solutions.
7. These containers are subjected to an automatic washing process in a machine that provides a pressurized water jet, this water is totally purified.
8. Once these containers leave the washing process, they enter a sterilization machine or dryer that will be in charge of sterilizing and drying the containers through the action of high temperatures.
9. Upon leaving the sterilization or drying machine, these containers enter an SCT Pharma oven, where the dehydrogenation process will be carried out, which is nothing more than the process that will allow us to reduce the total or almost total endotoxins. Bacterial (Pyrogens), in other words what we are looking for is to obtain blisters free of all microorganisms and undesirable pathogenic substances, we achieve this through processes that use high temperatures, such as those that have been previously exposed.
10. The resulting solution obtained in step 5. From the filter. You will now move on to a piece of equipment called a vial filling and sealing machine. The objective in this step is to dose the filtered solution as quickly as possible into the drinkable ampoules, taking care not to wet the neck of the ampoules. This machine will subsequently be responsible for sealing the valves, since it has a sealing system for this type of ampoules.
11. The next stage is the stage called labeling, stage in which the label is placed on the product including elementary information such as: Lot number, Dosage, Manufacturing Date, Expiration Date, etc.
12. In the final stage of our process, we find the storage of our finished product, under favorable conditions of temperature and humidity, characteristic of this type of product. The drinkable ampoules thus obtained according to the process described have an ambient storage stability of 2 years.

### Example 3.

Patients with coronavirus must undergo treatment for a period of 7 days and with a dosage of 3 drinkable ampoules per day, with a composition of ingredients per ampule of:

| | |
|---|---|
| Properly treated water | 19.5% |
| Non-hydrolyzed collagen | 35% |
| Dandelion Extract | 10% |
| Anamu extract | 8% |
| Epazote | 12% |
| Oregano | 10% |
| Star anise | 5% |
| Sodium benzoate | 0.25% |
| Potassium Sorbate | 0.25% |

### PROCEDURE FOR THE PREPARATION OF AN INTRAMUSCULAR INJECTABLE SOLUTION

The procedure for the preparation of an intramuscular injectable solution consists of the following steps:
1. On the first stage of the process, the different raw materials are received at the plant, which will go through an inspection, classification, and approval process to be used in the production of our final product, which will be an intramuscular injectable solution.
2. After the raw materials have been approved in the raw material reception area, they go to the pre-production area where they are prepared for entry into the production area. It is in this area where the shell is removed from those materials. Raw materials that require it, as well as the seeds, are cleaned and rinsed with treated water from the treatment plant.
3. When the raw materials leave this area, they enter the production area where the first equipment or unit operation they enter is a stainless-steel mixer with temperature control, which is fed with steam, which allows to achieve the increase of the temperature in it. In this mixer we add all the raw materials of our formula, except for the non-hydrolyzed collagen, the lidocaine, the preservative and the ascorbic acid, because these must be added at a temperature of 30°C to 40°C to avoid their degradation by high temperatures and then cannot have the expected effect on the final product.
4. The different raw materials are placed in this mixer, all mixed within it, they are subjected to a temperature of 96°C (205°F), followed by periods of agitation every 5 minutes, with the aim that at that temperature the water can absorb the properties and nutrients of each of the raw materials present in the mixer, this will allow us to obtain a well-charged liquid, rich in all the properties from the herbs, fruits and raw materials that were subjected in this part of the production process .
5. The resulting liquid that comes out of the mixer now goes to another piece of equipment called a pressure filter. It is in this equipment that even the smallest solid particle that may be mixed with the liquid extract is separated. The objective in this part of the process is to obtain a liquid free of solid particles. In this stage we will take the opportunity to add those pending products that we did not add in the previous stage due to the high temperatures, these are the non-hydrolyzed collagen, the preservative, the lidocaine and the ascorbic acid.
6. The container chosen to store the final product is the container: Glass Ampoule for Injectable Solutions.
7. These containers are subjected to an automatic washing process in a machine that provides a pressurized water jet, this water is totally purified.
8. Once these containers leave the washing process, they enter a sterilization machine or dryer that will be in charge of sterilizing and drying the containers through the action of high temperatures.
9. Upon leaving the sterilization or drying machine, these containers enter an SCT Pharma oven, where the depyrogenation process will be carried out, which is nothing more than the process that will allow us to reduce the total or almost total endotoxins. Bacterial (Pyrogens), in other words what we are looking for is to obtain blisters free of all microorganisms and undesirable pathogenic substances, we achieve this through processes that use high temperatures, such as those that have been previously exposed.
10. The resulting solution that we obtained in step 5, coming from the filter, will now pass to a piece of equipment called the Vial Filling and Sealing Machine. The objective in this step is to dose the filtered solution as quickly as possible into the glass ampoules, taking care always not to wet the neck of the blisters. This stage will end with the injection of inert gas. This machine will later be in charge of sealing the valves, since it has a sealing system for glass-type ampoules, which were the types of packaging chosen in our process.
11. Once we have the glass ampoules ready, containing the product inside and duly sealed, they are sterilized, this with the aim of eliminating any possible microorganism or contamination that may have remained on the outside of the container during the process, for which uses a sterilization equipment called an autoclave, the process that is carried out in this equipment is sterilization by moist heat, in which this equipment uses saturated water vapor, at a pressure of 15 pounds, which allows the chamber to reach a temperature of 121°C, in this equipment the ampoules will last about 15 minutes going through the sterilization process.
12. The next stage is the stage called labeling, stage in which the label is placed on the product including basic information such as: batch number, dose, route of administration, manufacturing date, expiration date. The ampoules of injectable solution thus obtained. The next stage is the stage called labeling, stage in which the label is placed on the product including basic information such as: Lot number, Dosage, Manufacturing Date, Expiration Date, etc.

The ampoules of injectable solution thus obtained according to the described process have an ambient storage stability of 2 years.

### Example 4.

Patients positive for coronavirus will undergo treatment for a period of 7 days with a dosage of 3 ampoules of intramuscular injectable solution per day. With a composition of ingredients per ampoule of:

| | | |
|---|---|---|
| Properly treated water | 18.5% | |
| Non-hydrolyzed collagen | 24% | |
| Dandelion | 13% | |
| Anamu | 18% | |
| Epazote | 8% | |
| Oregano | 8% | |
| Star anise | 8.51% | |
| Lidocaine | 1.8% | anesthetic |
| Benzoic Acid | 0.17% | preservative |
| Ascorbic acid | 0.02% | antioxidant |

According to a not-claimed embodiment, a group of 5 patients was taken and they were evaluated through a self-assessment of their condition before starting the plan and after 7 days of drinking two cups of tea a day, for this assessment they were asked to rate 1. On the 5th, his improvement with respect to his situation at the beginning of drinking the tea, the parameters to be assessed were muscle pain, dry cough, shortness of breath, diarrhea, tiredness, loss of the sense of smell and taste, body rash and fever. The following table shows the results of the self-assessment of their improvement with drinking the tea.

**Table No. 1 Tabulation of the self-assessment of a sample of 5 patients in the improvement of their individual coronavirus status with treatment for 7 days with the tea object of the present invention, assessment of the improvement with a weighting from 1 to 5.**

| Patient | Headaches and muscle aches | | Cough and shortness of breath | | Loss of smell and taste | | Diarrhea and fever | |
|---|---|---|---|---|---|---|---|---|
| | Before | Assessment | Before | Assessment | Before | Assessment | Before | Assessment |
| 1 | Yes | 5 | Yes | 5 | Yes | 5 | Yes | 5 |
| 2 | Yes | 5 | Yes | 5 | Yes | 5 | Yes | 5 |
| 3 | Yes | 4 | Yes | 5 | Yes | 4 | Yes | 5 |
| 4 | Yes | 5 | Yes | 5 | no | 0 | Yes | 5 |
| 5 | Yes | 5 | Yes | 5 | Yes | 4 | Yes | 5 |
| Average rating improvement | 4.80 | | 5 | | 4.50 | | 5 | |

The results obtained from the self-assessment that each patient who underwent the tea treatment where no other medication was taken for the relief of covid-19.

To be sure of its effectiveness, I was infected four times and the four times taking the formulation the symptoms disappeared with a proven improvement in 24 hours, the action of the formulation is one hundred percent effective, and does not carry any risk for the patient, with the advantage that it can be taken as prevention before being in places where there are people together without side effects, say an airplane; a restaurant; a cinema; since the virus would be inhibited by ingesting it. In 7 days, the person is recovered and it is a clear sign that the mechanism of action of these active ingredients disables the virus. Negative collateral reactions were manifested in none of the patients who received the treatment.

We are facing a simple but great solution to this pandemic, soap is the only thing that inhibits it, and drinking it is the only solution.

## Claims

1. Pharmaceutical composition for the treatment and prevention of coronavirus whose formulation contains as ingredients non-hydrolyzed collagen as a surfactant with degreasing properties in addition to natural plants in the form of lyophilized powders of aqueous extracts comprising Dandelion (Taraxacum officinale), Anamú (Petiveria Alliacea), Epazote (Dysphania ambrosioides), Oregano (Origanum vulgare), star anise (Lllicium verun), together with preservatives,
wherein the composition is **characterized by** the following list of ingredients:
| | |
|---|---|
| Non-hydrolyzed collagen | 1% up to 35% |
| Dandelion extract (Taraxacum officinale) | 5% up to 30% |
| Anamu extract (Anamau Petiveria) | 3% up to 20% |
| Epazote extract (Dysphania ambrosioides) | 3% up to 20% |
| Oregano extract (Origanum vulgare) | 5% up to 25% |
| Star anise extract (Lllicium verun) | 1% up to 15% |
| Sodium benzoate (Soda Benzoate) | 0.25% |
| Potassium sorbate (Potassium (2E,4E)-hexadienoate) | 0.25% |

2. Pharmaceutical composition for the treatment and prevention of coronavirus whose formulation contains as ingredients non-hydrolyzed collagen as a surfactant with degreasing properties in addition to natural plants in the form of lyophilized powders of aqueous extracts **characterized in that** it comprises Dandelion (Taraxacum officinale), Anamú (Petiveria Alliacea), Epazote (Dysphania ambrosioides), Oregano (Origanum vulgare), star anise (Lllicium verun), together with preservatives,
wherein the composition is in oral form and is formulated in a hard gelatin capsule comprising:
| | |
|---|---|
| Non-hydrolyzed collagen | 25% |
| Dandelion extract (Taraxacum officinale) | 20% |
| Anamu extract (Anamau Petiveria) | 10% |
| Epazote extract (Dysphania ambrosioides) | 15% |
| Oregano extract (Origanum vulgare) | 19.5% |
| Star anise extract (Lllicium verun) | 10% |
| Sodium benzoate (Soda Benzoate) | 0.25% |
| Potassium sorbate Potassium (2E,4E)-hexadienoate) | 0.25% |

3. Pharmaceutical composition for the treatment and prevention of coronavirus whose formulation contains as ingredients non-hydrolyzed collagen as a surfactant with degreasing properties in addition to natural plants in the form of lyophilized powders of aqueous extracts comprising Dandelion (Taraxacum officinale), Anamú (Petiveria Alliacea), Epazote (Dysphania ambrosioides), Oregano (Origanum vulgare), star anise (Lllicium verun), together with preservatives,
wherein the composition is in oral form and is formulated in drinkable ampoules, comprising:
| | |
|---|---|
| Properly treated water | 19.5% |
| Non-hydrolyzed collagen | 35% |
| Dandelion Extract (Taraxacum officinale) | 10% |
| Anamu Extract (Anamau Petiveria) | 8% |
| Epazote Extract (Dysphania ambrosioides) | 12% |
| Oregano extract (Origanum vulgare) | 10% |
| Star anise extract (Lllicium verun) | 5% |
| Sodium benzoate (Soda Benzoate) | 0.25% |
| Potassium sorbate Potassium (2E,4E)-hexadienoate) | 0.25% |

4. Pharmaceutical composition for the treatment and prevention of coronavirus whose formulation contains as ingredients non-hydrolyzed collagen as a surfactant with degreasing properties in addition to natural plants in the form of lyophilized powders of aqueous extracts comprising Dandelion (Taraxacum officinale), Anamú (Petiveria Alliacea), Epazote (Dysphania ambrosioides), Oregano (Origanum vulgare), star anise (Lllicium verun), together with preservatives,
wherein the composition is formulated in an intramuscular injectable solution, comprising:
| | | |
|---|---|---|
| Properly treated water | 18.5% | |
| Non-hydrolyzed collagen | 24% | |
| Dandelion extract (Taraxacum officinale) | 13% | |
| Anamu Extract (Anamau Petiveria) | 18% | |
| Epazote Extract (Dysphania ambrosioides) | 8% | |
| Oregano extract (Origanum vulgare) | 8% | |
| Star anise extract (Lllicium verun) | 8.51% | |
| Lidocaine2-(Diethylamino)-N-(2,6-dimethylphenyl) acetamide | 1.8% | anesthetic |
| Benzoic Acid (Benzenecarboxylic acid) | 0.17% | |
| Ascorbic acid (5R)-[(1S)-1,2-dihydroxyethyl] -3,4-dihydroxyfuran-2(5H)-one | 0.02% | |

5. Procedure for obtaining the pharmaceutical composition of claim 2 in the form of hard gelatin capsule capsules, **characterized by** the following steps:
I. Inspection, classification, and approval of the raw material.
II. Separation of shell and seeds to those raw materials that require it.
III. Mixing of the raw materials with temperature control at 96°C (205°F), and stirring every 5 minutes.
IV. Filtering and addition of preservatives.
V. Homogenized spraying of the active ingredients and preservatives.
VI. Encapsulating the obtained powder in capsules, in particular in gelatin capsules.

6. Procedure for obtaining the pharmaceutical composition of claim 3 in the form of drinkable ampoules, **characterized by** the following steps:
I. Inspection, classification, and approval of the raw material.
II. Separation of shell and seeds to those raw materials that require it.
III. Mixing of the raw materials with temperature control at 96°C (205°F) and stirring every 5 minutes.
IV. Filtering and addition of preservatives.
V. Filling the obtained product into an ampoule and sealing the ampoule.

7. Procedure for obtaining the pharmaceutical composition of claim 4 in the form of an intramuscular injectable solution, **characterized by** the following steps:
I. Inspection, classification, and approval of the raw material.
II. Separation of shell and seeds to those raw materials that require it.
III. Mixing of the raw materials with temperature control at 96°C (205°F) and stirring every 5 minutes.
IV. Filtering and addition of preservatives.
V. Filling the obtained product into an ampoule, followed by sealing the ampoule.

## Patentansprüche

1. Pharmazeutische Zusammensetzung zur Behandlung und Vorbeugung von Coronaviren, deren Formulierung als Inhaltsstoffe nicht-hydrolysiertes Kollagen als Tensid mit entfettenden Eigenschaften sowie natürliche Pflanzen in Form von gefriergetrockneten Pulvern aus wässrigen Extrakten enthält, welche aus Löwenzahn (Taraxacum officinale), Anamú (Petiveria Alliacea), Gänsefuß (Dysphania ambrosioides), Oregano (Origanum vulgare) und Sternanis (Illicium verum) sowie Konservierungsmittel bestehen,
wobei die Zusammensetzung durch die folgende Liste von Inhaltsstoffen gekennzeichnet ist:
| | |
|---|---|
| Nicht hydrolysiertes Kollagen | 1 % bis 35 % |
| Löwenzahn-Extrakt (Taraxacum officinale) | 5 % bis 30 % |
| Anamu-Extrakt (Anamau Petiveria) | 3 % bis 20 % |
| Gänsefuß-Extrakt (Dysphania ambrosioides) | 3 % bis 20 % |
| Oregano-Extrakt (Origanum vulgare) | 5 % bis 25 % |
| Sternanis-Extrakt (Illicium verum) | 1 % bis 15 % |
| Natriumbenzoat (Soda Benzoate) | 0,25 % |
| Kaliumsorbat (Kalium-(2E,4E)-hexadienoat) | 0,25 % |

2. Arzneimittelzusammensetzung zur Behandlung und Vorbeugung von Coronaviren, deren Formulierung als Inhaltsstoffe nicht-hydrolysiertes Kollagen als Tensid mit entfettenden Eigenschaften sowie natürliche Pflanzen in Form von gefriergetrockneten Pulvern aus wässrigen Extrakten enthält, **dadurch gekennzeichnet, dass** sie Löwenzahn (Taraxacum officinale), Anamú (Petiveria Alliacea), Gänsefuß (Dysphania ambrosioides), Oregano (Origanum vulgare) und Sternanis (Illicium verum) sowie Konservierungsmittel enthält,
wobei die Zusammensetzung in oraler Form vorliegt und in einer Hartgelatinekapsel enthalten ist, bestehend aus:
| | |
|---|---|
| nicht hydrolysiertes Kollagen | 25 % |
| Löwenzahn-Extrakt (Taraxacum officinale) | 20 % |
| Anamu-Extrakt (Anamau Petiveria) | 10 % |
| Gänsefuß-Extrakt (Dysphania ambrosioides) | 15 % |
| Oregano-Extrakt (Origanum vulgare) | 19,5 % |
| Sternanis-Extrakt (Illicium verum) | 10 % |
| Natriumbenzoat (Soda Benzoate) | 0,25 % |
| Kaliumsorbat Kalium (2E,4E)-hexadienoat) | 0,25 % |

3. Arzneimittelzusammensetzung zur Behandlung und Vorbeugung von Coronaviren, deren Formulierung als Inhaltsstoffe nicht-hydrolysiertes Kollagen als Tensid mit entfettenden Eigenschaften sowie natürliche Pflanzen in Form von gefriergetrockneten Pulvern aus wässrigen Extrakten enthält, bestehend aus Löwenzahn (Taraxacum officinale), Anamú (Petiveria Alliacea), Gänsefuß (Dysphania ambrosioides), Oregano (Origanum vulgare) und Sternanis (Illicium verum) sowie Konservierungsmittel enthält,
wobei die Zusammensetzung in oraler Form vorliegt und in Trinkampullen enthalten ist, bestehend aus:
| | |
|---|---|
| ordnungsgemäß aufbereitetes Wasser | 19,5 % |
| Nicht hydrolysiertes Kollagen | 35 % |
| Löwenzahn-Extrakt (Taraxacum officinale) | 10 % |
| Anamu-Extrakt (Anamau Petiveria) | 8 % |
| Gänsefuß-Extrakt (Dysphania ambrosioides) | 12 % |
| Oregano-Extrakt (Origanum vulgare) | 10 % |
| Sternanis-Extrakt (Illicium verum) | 5 |
| Natriumbenzoat (Soda Benzoate) | 0,25 % |
| Kaliumsorbat Kalium (2E,4E)-hexadienoat) | 0,25 % |

4. Arzneimittelzusammensetzung zur Behandlung und Vorbeugung von Coronaviren, deren Formulierung als Inhaltsstoffe nicht-hydrolysiertes Kollagen als Tensid mit entfettenden Eigenschaften sowie natürliche Pflanzen in Form von gefriergetrockneten Pulvern aus wässrigen Extrakten enthält, bestehend aus Löwenzahn (Taraxacum officinale), Anamú (Petiveria Alliacea), Gänsefuß (Dysphania ambrosioides), Oregano (Origanum vulgare) und Sternanis (Illicium verum) sowie Konservierungsmittel enthält,
wobei die Zusammensetzung in einer intramuskulär injizierbaren Lösung enthalten ist, bestehend aus:
| | |
|---|---|
| ordnungsgemäß aufbereitetem Wasser | 18,5 % |
| Nicht hydrolysiertes Kollagen | 24 % |
| Löwenzahn-Extrakt (Taraxacum officinale) | 13 |
| Anamu-Extrakt (Anamau Petiveria) | 18 |
| Gänsefuß-Extrakt (Dysphania ambrosioides) | 8% |
| Oregano-Extrakt (Origanum vulgare) | 8 |
| Sternanis-Extrakt (Illicium verum) | 8,51 % |
| Lidocain 2-(Diethylamino)-N-(2,6-Dimethylphenyl)acetamid | 1,8 % |
| Anästhetikum Benzoesäure (Benzolcarbonsäure) | 0,17 % |
| Ascorbinsäure (5R)-[(1S)-1,2-Dihydroxyethyl] -3,4-dihydroxyfuran-2(5H)-on | 0,02 |

5. Verfahren zur Herstellung der pharmazeutischen Zusammensetzung nach Anspruch 2 in Form von Hartgelatinekapseln, **gekennzeichnet durch** die folgenden Schritte:
I. Prüfung, Klassifizierung und Freigabe des Rohmaterials.
II. Trennung von Schalen und Samen bei denjenigen Rohstoffen, bei denen dies erforderlich ist.
III. Mischen der Rohstoffe bei einer Temperatur von 96 °C (205 °F) unter Rühren alle 5 Minuten.
IV. Filtrieren und Zugabe von Konservierungsmitteln.
V. Homogenisierte Zerstäubung der Wirkstoffe und Konservierungsmittel.
VI. Einkapseln des gewonnenen Pulvers in Kapseln, insbesondere in Gelatinekapseln.

6. Verfahren zur Herstellung der pharmazeutischen Zusammensetzung nach Anspruch 3 in Form von Trinkampullen, **gekennzeichnet durch** die folgenden Schritte:
I. Prüfung, Klassifizierung und Freigabe des Rohmaterials.
II. Trennung von Schalen und Samen bei denjenigen Rohstoffen, bei denen dies erforderlich ist.
III. Mischen der Rohstoffe bei einer Temperatur von 96 °C (205 °F) unter Rühren alle 5 Minuten.
IV. Filtrieren und Zugabe von Konservierungsmitteln.
V. Abfüllen des erhaltenen Produkts in eine Ampulle und Abdichten der Ampulle.

7. Verfahren zur Herstellung der pharmazeutischen Zusammensetzung nach Anspruch 4 in Form einer intramuskulär injizierbaren Lösung, **gekennzeichnet durch** die folgenden Schritte:
I. Prüfung, Klassifizierung und Freigabe des Rohmaterials.
II. Trennung von Schalen und Samen bei denjenigen Rohstoffen, bei denen dies erforderlich ist.
III. Mischen der Rohstoffe bei einer Temperatur von 96 °C (205 °F) unter Rühren alle 5 Minuten.
IV. Filtrieren und Zugabe von Konservierungsmitteln.
V. Abfüllen des erhaltenen Produkts in eine Ampulle, gefolgt vom Abdichten der Ampulle.

## Revendications

1. Composition pharmaceutique pour le traitement et la prévention du coronavirus dont la formulation contient comme ingrédients du collagène non hydrolysé en tant que surfactant aux propriétés dégraissantes, ainsi que des plantes naturelles sous forme de poudres lyophilisées d'extraits aqueux comprenant du pissenlit (Taraxacum officinale), de l'anamú (Petiveria Alliacea), de l'épazote (Dysphania ambrosioides), de l'origan (Origanum vulgare), de l'anis étoilé (Lllicium verun), ainsi que des conservateurs,
dans laquelle la composition est **caractérisée par** la liste suivante d'ingrédients :
| | |
|---|---|
| Collagène non hydrolysé | de 1 % jusqu'à 35 % |
| Extrait de pissenlit (Taraxacum officinale) | de 5 % jusqu'à 30 % |
| Extrait d'anamú (Anamau Petiveria) | de 3 % jusqu'à 20 % |
| Extrait d'épazote (Dysphania ambrosioides) | de 3 % jusqu'à 20 % |
| Extrait d'origan (Origanum vulgare) | de 5 % jusqu'à 25 % |
| Extrait d'anis étoilé (Lllicium verun) | de 1 % jusqu'à 15 % |
| Benzoate de sodium (Soda Benzoate) | 0,25 % |
| Sorbate de potassium (hexadiénoate de potassium (2E,4E)) | 0,25 % |

2. Composition pharmaceutique pour le traitement et la prévention du coronavirus dont la formulation contient comme ingrédients du collagène non hydrolysé en tant que surfactant aux propriétés dégraissantes, ainsi que des plantes naturelles sous forme de poudres lyophilisées d'extraits aqueux **caractérisés en ce qu'**ils comprennent du pissenlit (Taraxacum officinale), de l'anamú (Petiveria Alliacea), de l'épazote (Dysphania ambrosioides), de l'origan (Origanum vulgare), de l'anis étoilé (Lllicium verun), ainsi que des conservateurs,
dans lequel la composition est sous forme orale et est formulée dans une capsule de gélatine dure comprenant :
| | |
|---|---|
| Collagène non hydrolysé | 25 % |
| Extrait de pissenlit (Taraxacum officinale) | 20 % |
| Extrait d'anamú (Anamau Petiveria) | 10 % |
| Extrait d'épazote (Dysphania ambrosioides) | 15 % |
| Extrait d'origan (Origanum vulgare) | 19,5 % |
| Extrait d'anis étoilé (Lllicium verun) | 10 % |
| Benzoate de sodium (Soda Benzoate) | 0,25 % |
| Sorbate de potassium (hexadiénoate de potassium-(2E,4E)) | 0,25 % |

3. Composition pharmaceutique pour le traitement et la prévention du coronavirus dont la formulation contient comme ingrédients du collagène non hydrolysé en tant que surfactant aux propriétés dégraissantes, ainsi que des plantes naturelles sous forme de poudres lyophilisées d'extraits aqueux comprenant du pissenlit (Taraxacum officinale), de l'anamú (Petiveria Alliacea), de l'épazote (Dysphania ambrosioides), de l'origan (Origanum vulgare), de l'anis étoilé (Lllicium verun), ainsi que des conservateurs,
dans laquelle la composition est sous forme orale et est formulée dans des ampoules buvables, comprenant :
| | |
|---|---|
| Eau correctement traitée | 19,5 % |
| Collagène non hydrolysé | 35 % |
| Extrait de pissenlit (Taraxacum officinale) | 10 % |
| Extrait d'Anamú (Anamau Petiveria) | 8 % |
| Extrait d'épazote (Dysphania ambrosioides) | 12 % |
| Extrait d'origan (Origanum vulgare) | 10 % |
| Extrait d'anis étoilé (Lllicium verun) | 5 % |
| Benzoate de sodium (Soda Benzoate) | 0,25 % |
| Sorbate de potassium (hexadiénoate de potassium-(2E,4E)) | 0,25 % |

4. Composition pharmaceutique pour le traitement et la prévention du coronavirus dont la formulation contient comme ingrédients du collagène non hydrolysé en tant que surfactant aux propriétés dégraissantes, ainsi que des plantes naturelles sous forme de poudres lyophilisées d'extraits aqueux comprenant du pissenlit (Taraxacum officinale), de l'anamú (Petiveria Alliacea), de l'épazote (Dysphania ambrosioides), de l'origan (Origanum vulgare), de l'anis étoilé (Lllicium verun), ainsi que des conservateurs,
dans laquelle la composition est formulée dans une solution injectable intramusculaire, comprenant :
| | | |
|---|---|---|
| Eau correctement traitée | 18,5 % | |
| Collagène non hydrolysé | 24 % | |
| Extrait de pissenlit (Taraxacum officinale) | 13 % | |
| Extrait d'Anamú (Anamau Petiveria) | 18 % | |
| Extrait d'épazote (Dysphania ambrosioides) | 8 % | |
| Extrait d'origan (Origanum vulgare) | 8 % | |
| Extrait d'anis étoilé (Lllicium verun) | 8,51 % | |
| Lidocaïne2-(Diéthylamino)-N-(2,6-diméthylphényl) | | anesthésique |
| acétamide | 1,8 % | |
| Acide benzoïque (acide benzène-carboxylique) | 0,17 % | |
| Acide ascorbique (5R)-[(1S)-1,2-dihydroxyéthyl]-3,4-dihydroxyfuran-2(5H)-one | 0,02 % | |

5. Procédé d'obtention de la composition pharmaceutique selon la revendication 2 sous forme de gélules de gélatine dure, **caractérisé par** les étapes suivantes :
I. Inspection, classification et approbation de la matière première.
II. Séparation des coquilles et des graines pour les matières premières qui le nécessitent.
III. Mélange des matières premières avec contrôle de la température à 96°C (205°F), et agitation toutes les 5 minutes.
IV. Filtration et ajout de conservateurs.
V. Pulvérisation homogénéisée des ingrédients actifs et des conservateurs.
VI. Encapsulation de la poudre obtenue dans des gélules, en particulier dans des gélules de gélatine.

6. Procédé d'obtention de la composition pharmaceutique selon la revendication 3 sous forme d'ampoules buvables, **caractérisé par** les étapes suivantes :
I. Inspection, classification et approbation de la matière première.
II. Séparation des coquilles et des graines pour les matières premières qui le nécessitent.
III. Mélange des matières premières avec contrôle de la température à 96°C (205°F) et agitation toutes les 5 minutes.
IV. Filtration et ajout de conservateurs.
V. Remplissage du produit obtenu dans une ampoule et scellement de l'ampoule.

7. Procédé d'obtention de la composition pharmaceutique selon la revendication 4 sous forme de solution injectable intramusculaire, **caractérisé par** les étapes suivantes :
I. Inspection, classification et approbation de la matière première.
II. Séparation des coquilles et des graines pour les matières premières qui le nécessitent.
III. Mélange des matières premières avec contrôle de la température à 96°C (205°F) et agitation toutes les 5 minutes.
IV. Filtration et ajout de conservateurs.
V. Remplissage du produit obtenu dans une ampoule, suivi du scellement de l'ampoule.
